# EUROPEAN PATENT APPLICATION

(11) **EP 4 692 107 A1**
(43) Date of publication of application: **11.02.2026**
(21) Application number: 24785164.5
(22) Date of filing: 02.04.2024
(51) Int. Cl.: C07K 7/06, A61K 8/64, A61Q 19/00

(54) **PEPTIDE HAVING SKIN CONDITION IMPROVEMENT ACTIVITY AND USE THEREOF**

(30) Priority: 05.04.2023 KR 20230044929
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR)
(74) Representative: Potter Clarkson
(86) International application number: PCT/KR2024/004241
(87) International publication number: WO 2024/210459

(57) **Abstract**

The present application relates to a peptide having the activity of improving the state of skin, and uses thereof, and provides a peptide consisting of the amino acid sequence of SEQ ID NO: 1, and a cosmetic composition for improving skin condition including the peptide as an active ingredient.

## Description

### Technical Field

The present disclosure relates to peptides having the activity of improving the state of skin, and uses thereof.

### Background Art

Human skin is constantly subjected to changes, the most representative of which is the deterioration of skin function and decrease in visual beauty due to aging. Aging causes the formation of wrinkles in the skin, and typical factors for wrinkle formation include exposure to ultraviolet rays and decreased collagen biosynthesis, etc. Skin aging may be broadly categorized into intrinsic aging, which is caused by genetic factors, and extrinsic aging, which is caused by external environmental factors such as solar radiation. Among these, in the case of extrinsic aging, it is known that aging may be prevented, treated or delayed by removal of reactive oxygen species, proliferation of fibroblasts, promotion of collagen biosynthesis, etc.

On the other hand, collagen, a major component of the extracellular matrix, is the main matrix protein produced by fibroblasts in the skin. Collagen forms the majority of organic matter in skin, tendons, bones, and teeth, with its content being particularly high in bone and skin (dermis). This collagen reduces with age and photoaging due to ultraviolet irradiation, which is known to be closely related to the formation of wrinkles in the skin. Furthermore, collagen plays an important role in wound healing, and by stimulating the synthesis of collagen in damaged epithelium, wounds may be repaired quickly and without scarring. In addition, it has been reported that as the biosynthesis of collagen is promoted, the density of the basal layer, etc. increases, the melanin pigment concentration per unit skin density decreases, and the effect of brightening skin tone may be expected.

Against this technical background, multifaceted studies are being conducted to improve the state of skin through mechanisms such as promotion of collagen biosynthesis and enhancement of proliferation and activity of fibroblasts (Korean Registered Patent No. 10-1043081), but these are still insufficient.

### Disclosure of Invention

### Technical Problem

One aspect is to provide a peptide consisting of the amino acid sequence of SEQ ID NO: 1.

Another aspect is to provide a cosmetic composition for improving skin condition, including the above peptide as an active ingredient.

Other objectives and advantages of the present application will become apparent from the following detailed description, together with the appended claims and drawings. Any matter not described herein will be sufficiently recognized and inferred by those skilled in the art of the present application or a similar art, and the description is hereby omitted.

### Solution to Problem

Each of the descriptions and embodiments disclosed in the present application may be applied to other descriptions and embodiments. Accordingly, all combinations of the various elements disclosed in the present application fall within the scope of the present application. Furthermore, the scope of the present application is not to be considered limited by the specific description set forth below.

One aspect provides a peptide consisting of the amino acid sequence of SEQ ID NO: 1.

As used herein, the term "peptide" may refer to a linear molecule formed by combining amino acid residues to each other by peptide bonds. The above peptide may be prepared according to chemical synthesis methods known in the art, particularly solidphase synthesis techniques (Merrifield, J. Amer. Chem. Soc. 85:2149-54 (1963)) or liquidphase synthesis techniques (US Patent No. 5,516,891).

As a result of diligent efforts to develop a peptide with biologically effective activity, the inventors of the present disclosure have identified a peptide consisting of the amino acid sequence of SEQ ID NO: 1. Here, the biologically effective activity may be one or more selected from: (a) promotion of proliferation of human dermal fibroblasts; and (b) increased expression of a dermal extracellular matrix selected from collagen type 1, fibronectin, or elastin. Therefore, the above peptide may be used for improving skin condition.

The peptide may have a protecting group bound to the N- or C-terminus of the peptide to acquire chemical stability, enhanced pharmacological properties (half-life, absorption, titer, effect, etc.), altered specificity (for example, broad spectrum of biological activity), or reduced antigenicity. Accordingly, the peptide may be used to include both the peptide itself and a protected derivative thereof in which a protecting group is introduced. In an embodiment, an N-terminus of the peptide may be bound with any one protecting group selected from the group consisting of an acetyl group, a fluoreonylmethoxycarbonyl group, a formyl group, a palmitoyl group, myristyl group, stearyl group, butoxycarbonyl group (Boc), allyloxycarbonyl group (Alloc), and polyethylene glycol (PEG); and/or the C-terminus of the peptide may be bound with any one protecting group selected from the group consisting of an amino group(-NH₂), a tertiary alkyl group, and an azide (-NHNH₂). Additionally, the peptide may optionally further include a targeting sequence, a tag, labeled residues, or an amino acid sequence prepared for the specific purpose of increasing the half-life or peptide stability.

The above peptide is artificially synthesized, or non-naturally occurring or engineered, and the "non-naturally occurring or engineered" refers to a state that is not in its natural state, but is created by artificial modification. Here, the artificial modification may include artificially synthesizing an amino acid sequence by mimicking a plurality of amino acid structures, or manipulating it to acquire chemical stability, enhanced pharmacological properties, altered specificity, or reduced antigenicity as described above.

As used herein, the term "stability" may refer to not only in vivo stability, which protects the peptide from attack by proteolytic enzymes in vivo, but also storage stability (for example, room temperature storage stability).

Another aspect provides a cosmetic composition for improving skin condition including, as an active ingredient a peptide consisting of the amino acid sequence of SEQ ID NO: 1.

In the above description of the peptide, the terms or elements referred to are the same as those already mentioned.

As used herein, the term "improve" may refer to any act that at least reduces a parameter associated with the alleviation or treatment of a condition, for example the severity of a symptom.

As used herein, the term "improving skin condition" may comprehensively refer to the process or effect of treating, reducing, or alleviating skin damage, etc., caused by endogenous or exogenous factors of the skin, such as for example, the term may be interpreted as showing improvement of wrinkles, improvement of skin elasticity, or wound recovery, but is not limited thereto.

As used herein, "improving wrinkles," "improving skin elasticity," and "wound recovery" may refer to all actions that increases the total amount of extracellular matrix factors, including, promotion of collagen synthesis, etc.

Although prior functional peptides have effective biological activity, they have shown disadvantages such as not being able to effectively enter target tissue or cell due to the size of the peptide itself, or disappearing from the body in a short period of time due to their short half-life. In contrast, the cosmetic composition according to an example includes, as an active ingredient, a peptide consisting of 10 or fewer amino acids, and accordingly, the penetration rate, etc., of the active ingredient into the skin is very excellent, and skin condition may be effectively improved, for example, when applied topically to the skin.

According to an example, the peptide showed the effect of promoting human dermal fibroblast proliferation; and increasing the expression of dermal extracellular matrix selected from collagen type 1, fibronectin, or elastin. Thus, the peptide may be utilized as an active ingredient in a cosmetic composition for improving skin condition.

The cosmetic composition may include, a cosmetically effective amount of the peptide; and/or a cosmetically acceptable carrier, but is not limited thereto.

As used herein, the term "cosmetically effective amount" may refer to an amount sufficient to achieve skin condition improvement effect of the cosmetic composition.

The weight ratio between the peptide and a cosmetically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

The cosmetic composition may be prepared in any formulation commonly prepared in the art, for example, may be formulated as a solution, a suspension, an emulsion, a paste, a gel, a cream, a lotion, a powder, a soap, a surfactant-containing cleanser, an oil, a powder foundation, an emulsion foundation, a wax foundation, and a spray, etc., but is not limited thereto. For example, the cosmetic composition may be prepared in the form of a softening lotion, nutrition lotion, nutrition cream, massage cream, essence, eye cream, cleansing cream, cleansing foam, cleansing water, mask, spray or powder.

If the formulation of the cosmetic composition is a paste, cream or gel, the carrier component may be an animal oil, vegetable oil, wax, paraffin, starch, tragacanth, cellulose derivative, polyethylene glycol, silicone, bentonite, silica, talc or zinc oxide, etc.

If the formulation of the cosmetic composition is a powder or spray, lactose, talc, silica, aluminum hydroxide, calcium silicate or polyamide powder may be used as a carrier component and, for example, if the formulation is a spray, it may additionally include a propellant such as chlorofluorohydrocarbon, propane/butane or dimethyl ether.

If the formulation of the cosmetic composition is a solution or emulsion, a solvent, solubilizer or emulsifier is used as a carrier component and may include, for example, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol or fatty acid esters of sorbitan.

If the formulation of the cosmetic composition is a suspension, the carrier component may include a liquid diluent such as water, ethanol or propylene glycol, a suspending agent such as ethoxylated isostearyl alcohol, polyoxyethylene sorbitol esters and polyoxyethylene sorbitan esters, microcrystalline cellulose, aluminum methahydroxide, bentonite, agar or tragacanth, etc.

If the formulation of the cosmetic composition is a surfactant-containing cleanser, the carrier component may include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, lanolin derivative, or ethoxylated glycerol fatty acid ester, etc.

The peptide may be incorporated into a nanosome or a nanoparticle to further improve skin penetration or stability issues. For example, the nanosome may be prepared by a microfluidizer using lecithin as a raw material, and may be incorporated in lecithin particles. Any method of preparing the nanosome may be used as long as it is known. The size of the nanosome particle is preferably 30 to 200 nm. If the size of the nanosome particle is less than 30 nm, skin penetration may progress very quickly, resulting in skin side effects, and if the size is greater than 200 nm, skin penetration may not be easy, making it difficult to achieve the effect of using the nanosome structure.

The ingredients included in the cosmetic composition include, in addition to peptides and carrier components as active ingredients, components commonly used in cosmetic compositions, and may include, for example, common adjuvants such as an antioxidant, stabilizer, solubilizer, vitamin, pigment, and fragrance.

The content of the peptide as an active ingredient contained in the cosmetic composition may be selected appropriately without limitation depending on the form of the product, the desired use, etc., and may be added, for example, from 0.01 to 15 wt% of the total weight of the cosmetic composition. Additionally, for example, the cosmetic composition may include the peptide in an amount of from 1.0 wt% to 3.0 wt%, preferably from 2.0 wt% to 3.0 wt%, based on the total weight.

Another aspect provides a method of improving skin condition, including applying a cosmetic composition including a peptide consisting of the amino acid sequence of SEQ ID NO: 1 as an active ingredient to the skin of a subject.

In the above description of the cosmetic composition, the terms or elements referred to are the same as those already mentioned.

As used herein, the terms "applying," "administering," and "applying" are used interchangeably and may refer to causing at least partial localization of a composition according to an example to a desired site, or to placing a composition according to an example into a subject by route of administration.

Another aspect provides a pharmaceutical composition for improving skin condition including, as an active ingredient, a peptide consisting of the amino acid sequence of SEQ ID NO: 1.

In the above description of the peptide, the terms or elements referred to are the same as those already mentioned.

According to an example, the peptide showed the effect of promoting human dermal fibroblast proliferation; and increasing the expression of dermal extracellular matrix selected from collagen type 1, fibronectin, or elastin. Thus, the peptide may be utilized as an active ingredient in a pharmaceutical composition for improving skin condition.

The pharmaceutical composition may include, a pharmaceutically effective amount of the peptide; and/or a pharmaceutically acceptable carrier, but is not limited thereto.

As used herein, the term "pharmaceutically effective amount" refers to an amount sufficient to achieve the efficacy of the pharmaceutical composition in preventing or treating skin diseases.

The pharmaceutically acceptable carrier is commonly used in formulations and include, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, etc., but is not limited thereto. Suitable pharmaceutically acceptable carriers and formulations are described in detail in Remington's Pharmaceutical Sciences (19th ed., 1995).

The weight ratio between the peptide and a pharmaceutically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

In addition to the above ingredients, the pharmaceutical composition may additionally include, lubricants, humectant, sweetener, flavoring agent, emulsifier, suspending agent, preservative, etc., but is not limited thereto.

The pharmaceutical composition may be administered orally or parenterally, preferably parenterally, and if parenterally administered, may be administered by, but not limited to, intramuscular injection, intravenous injection, subcutaneous injection, intraperitoneal injection, topical administration, transdermal administration, etc.

The dosage of the pharmaceutical composition may be, but is not limited to, 0.0001 to 1000 µg (micrograms), 0.001 to 1000 µg, 0.01 to 1000 µg, 0.1 to 1000 µg, or 1.0 to 1000 µg per day, and may be varied by factors such as method of formulation, mode of administration, patient age, weight, sex, medical condition, food, time of administration, route of administration, rate of excretion, and response sensitivity.

The pharmaceutical composition may be formulated, with a pharmaceutically acceptable carrier and/or excipient, according to a method that could be readily practiced by a person skilled in the art to which the present disclosure belongs, and the composition may be prepared into a unit dosage form or may be contained in a multi-dose container.

The formulation may be in the form of a solution, suspension or emulsion in an oil or aqueous medium, or in the form of an extract, powder, granule, tablet or capsule, and may additionally include a dispersant and/or stabilizer.

Another aspect provides a food composition for improving the state of the skin including, as an active ingredient, a peptide consisting of the amino acid sequence of SEQ ID NO: 1.

In the above description of the peptide, the terms or elements referred to are the same as those already mentioned.

The content of the peptide as an active ingredient contained in the food composition may be appropriately selected without limitation depending on the form of the food, the desired use, etc., and may be added, for example, from 0.01 to 15 wt% of the total weight of the food. Additionally, for example, the health drink composition may be added at a rate of 0.02 to 10 g, preferably 0.3 to 1 g, based on 100 ml.

### Advantageous Effects of Invention

According to an aspect, the peptide may be applied to improve skin conditions including wrinkle improvement, skin elasticity improvement, and wound healing, etc., by promoting the proliferation of human dermal fibroblasts or increasing the expression of dermal extracellular matrix such as collagen, fibronectin, or elastin.

### Brief Description of Drawings

FIG. 1 shows the results of measuring the fibroblast cell growth promotion effect of a peptide consisting of the amino acid sequence of SEQ ID NO: 1.
FIG. 2 shows the results of measuring the effect of a peptide consisting of the amino acid sequence of SEQ ID NO: 1 on increasing the expression of skin extracellular matrix.

### Mode for the Invention

Hereinafter, the present disclosure will be described in more detail with reference to examples. However, these examples are intended to illustrate the present disclosure by way of example and the scope of the present disclosure is not limited to these examples.

### Example 1: Synthesis of Peptides

A peptide with the amino acid sequence of SEQ ID NO: 1 listed in Table 1 below was synthesized using an automated peptide synthesizer (Milligen 9050, Millipore, USA), and the synthesized peptide was purified by C18 reversed-phase high performance liquid chromatography (HPLC) (Waters Associates, USA). The column used was an ACQUITY UPLC BEH300 C18 (2.1 mm X 100 mm, 1.7 µm, Waters Co, USA).

**[Table 1]**

| SEQ ID NO: | Sequence (N terminal → C terminal) |
|---|---|
| 1 | AERRREKCRC |

### Example 2: Confirmation of Fibroblast Proliferation Effect

To confirm the effect of a peptide consisting of the amino acid sequence of SEQ ID NO: 1 on promoting the proliferation of Fibroblasts. Specifically, human dermal fibroblasts were seeded in a 96-well plate at a density of 5x10³ cells/well and cultured for 24 hours. After replacing with serum-free mesenchymal stem cell medium, the cells were cultured for 24 hours. The above peptide was treated at concentrations of 10 µM, 50 µM, or 100 µM, respectively, and incubated at 37 °C for 72 hours. As a positive control group, 50 nM of human recombinant FGF protein was treated. After completion of culturing, the culture supernatant was removed and the cells were fixed using ethanol. After washing with PBS, staining was performed by treating with SRB solution. After washing with 1 % acetic acid, cells were observed under a microscope and destained by treating with 20 mM tris solution. Cell viability was measured by measuring absorbance at a wavelength of 560 nm using a spectrophotometer (SpectraMax iD3, Molecular Devices (USA, CA)).

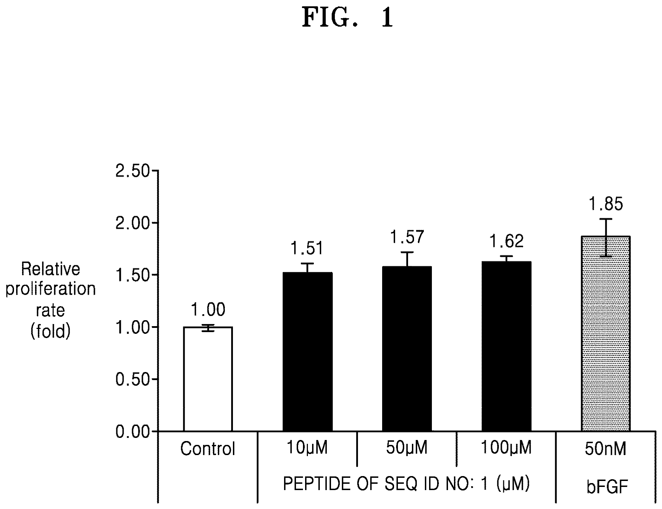

The results are illustrated in FIG. 1. From the results of FIG. 1, it may be confirmed that proliferation was induced in a concentration-dependent manner in the peptide treatment group, increasing by up to 1.62 times.

### Example 3: Confirmation of Effect of Increasing Extracellular Matrix Expression

To confirm the effect of a peptide consisting of the amino acid sequence of SEQ ID NO: 1 on increasing the expression of the dermal extracellular matrix. Specifically, human dermal fibroblasts were seeded in a 6-well plate at a density of 3x10⁵ cells/well and cultured for 24 hours. After replacing with serum-free mesenchymal stem cell medium, the cells were cultured for 24 hours. A peptide was treated at concentrations of 10 µM, 50 µM, or 100 µM, respectively, and incubated at 37 °C for 48 hours. Human recombinant FGF protein was treated at 100 nM. After the culture was completed, the culture supernatant was removed and fixed with 4 % paraformaldehyde for 30 minutes. After washing with PBS, the cells were reacted with 0.5 % Triton X-100 for 15 minutes. After reacting with 3 % BSA for 1 hour, primary antibodies against collagen type 1, fibronectin, and elastin were diluted 1:100 and reacted overnight at 4 °C. Secondary antibody-FITC was diluted 1:500 and reacted at room temperature for 2 hours, followed by DAPI staining and mounting. The expression images of each marker were observed using a confocal microscope (Leica, Germany). Green fluorescence intensity was measured using Image J.

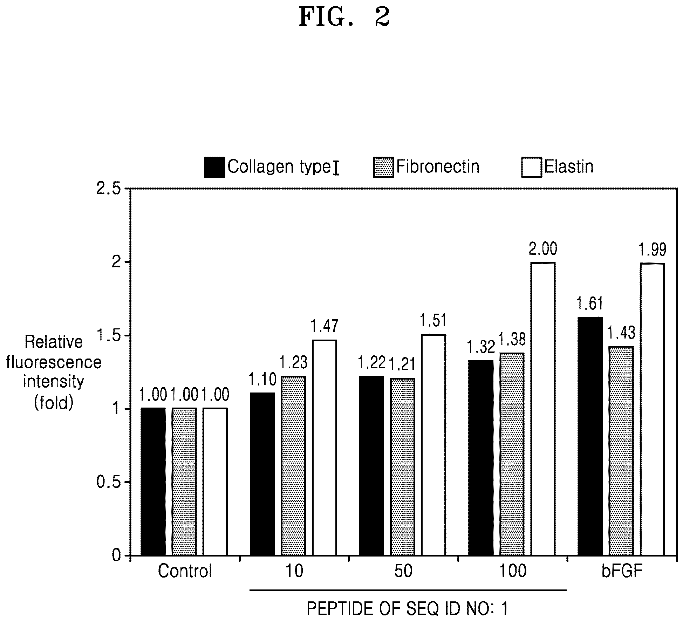

The results are illustrated in FIG. 2. From the results in Fig. 2, it may be confirmed that the expression of collagen type 1, fibronectin, and elastin, which are dermal extracellular matrix, increases when peptide is treated. For collagen, expression was confirmed to increase by up to 1.32 times compared to the control group, for fibronectin by up to 1.38 times, and for elastin by up to 2 times.

### Formulation Example 1. Preparation of Peptide Nanosomes

50 mg of the peptide of Example 1 above was dissolved in 500 ml of distilled water by sufficient stirring. The composite solution was mixed with 5 g of lecithin, 0.3 ml of sodium oleate, 50 ml of ethanol, and a small amount of oil, and the total amount was adjusted to 1 L with distilled water, and the mixture was then emulsified using a mat high pressure to produce peptide nanosomes with a size of about 100 nm.

### Formulation Example 2. Softening Lotion

A softening lotion including the peptide according to an example and consisting of the following composition was prepared using a method known in the art.

**[Table 2]**

| **Component** | **Content (wt%)** |
|---|---|
| Peptide | 1.0 |
| 1,3-Butylene glycol | 6 |
| Glycerin | 4 |
| PEG 1500 | 1 |
| Sodium hyaluronate | 1 |
| Polysorbate 20 | 0.5 |
| Ethanol | 8 |
| Benzophenone-9 | 0.05 |
| Preservatives, colorants | As required |
| Fragrance | As required |
| Purified water | Remainder |
| Total | 100 |

### Formulation Example 3. Nutrition Cream

A nutrition cream including the peptide according to an example and consisting of the following composition was prepared using a method known in the art.

**[Table 3]**

| **Component** | **Content (wt%)** |
|---|---|
| Peptide | 2.5 |
| Meadowfoam oil | 3 |
| Cetearyl alcohol | 1.5 |
| Stearic acid | 1.5 |
| Glyceryl stearate | 1.5 |
| Liquid paraffin | 10 |
| Beeswax | 2 |
| Polysorbate 60 | 0.6 |
| Sorbitan sesquioleate | 2.5 |
| Squalane | 3 |
| 1,3-Butylene glycol | 3 |
| Glycerin | 5 |
| Triethanolamine | 0.5 |
| Tocopheryl acetate | 0.5 |
| Preservatives, colorants | As required |
| Fragrance | As required |
| Purified water | Remainder |
| Total | 100 |

### Formulation Example 4. Nutrition Lotion

A nutrition lotion including the peptide according to an example and consisting of the following composition was prepared using a method known in the art.

**[Table 4]**

| **Component** | **Content (wt%)** |
|---|---|
| Peptide | 3.0 |
| 1,3-Butylene glycol | 4 |
| Glycerin | 4 |
| Cetearyl alcohol | 0.8 |
| Glyceryl stearate | 1 |
| Triethanolamine | 0.13 |
| Tocopheryl acetate | 0.3 |
| Liquid paraffin | 5 |
| Squalane | 3 |
| Oils | 2 |
| Polysorbate 60 | 1.5 |
| Sorbitan sesquioleate | 0.5 |
| Carboxyvinyl polymer | 1 |
| Preservatives, colorants | As required |
| Fragrance | As required |
| Purified water | Remainder |
| Total | 100 |

### Formulation Example 5. Essence

An essence including the peptide according to an example and consisting of the following composition was prepared using a method known in the art.

**[Table 5]**

| **Component** | **Content (wt%)** |
|---|---|
| Peptide | 2.0 |
| Glycerin | 10 |
| 1,3-Butylene glycol | 5 |
| PEG 1500 | 2 |
| Allantoin | 0.1 |
| DL-Panthenol | 0.3 |
| EDTA-2Na | 0.02 |
| Hydroxyethyl cellulose | 0.1 |
| Sodium hyaluronate | 8 |
| Carboxyvinyl polymer | 0.2 |
| Triethanolamine | 0.18 |
| Octyldodeceth-16 | 0.4 |
| Ethanol | 6 |
| Preservatives, colorants | As required |
| Fragrance | As required |
| Purified water | Remainder |
| Total | 100 |

The foregoing description of the present disclosure is for illustrative purposes only, and one that has ordinary skill in the art to which the present disclosure belongs will understand that it may be readily adapted to other specific forms without altering the technical ideas or essential features of the present disclosure. Therefore, the examples described above should be understood in all respects as illustrative and not restrictive.

## Claims

1. A peptide consisting of the amino acid sequence of SEQ ID NO: 1.

2. The peptide of claim 1, wherein an N-terminus of the peptide is bound to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG).

3. The peptide of claim 1, wherein a C-terminus of the peptide is bound to any one protecting group selected from the group consisting of an amino group (-NH₂), a tertiary alkyl group, and azide (-NHNH₂).

4. The peptide of claim 1, wherein the peptide exhibits any one or more characteristics selected from the following:
(a) promotion of proliferation of human dermal fibroblasts; and
(b) increased expression of a skin extracellular matrix selected from collagen type 1, fibronectin, or elastin.

5. A cosmetic composition for improving a state of skin, comprising, as an active ingredient, a peptide according to any one of claims 1 to 4.

6. The composition of claim 5, wherein the improvement of the state of skin is improvement in wrinkles, improvement in skin elasticity, or wound healing.
